Europäisches Patentamt

European Patent Office

Office européen des brevets

(19)

(11) Numéro de publication: **0 203 863 B1**

(12) **FASCICULE DE BREVET EUROPEEN**

(45) Date de publication de fascicule du brevet:
**02.05.91**

(51) Int. Cl.5: **C12N 15/74**, C12N 15/52,
A01N 63/00

(21) Numéro de dépôt: **86401104.4**

(22) Date de dépôt: **26.05.86**

(54) **Vecteurs de transformation des micrcorganismes à action rhizospherique favorable, souches transformées et applications agronomiques.**

(30) Priorité: **31.05.85 FR 8508237**

(43) Date de publication de la demande:
**03.12.86 Bulletin 86/49**

(45) Mention de la délivrance du brevet:
**02.05.91 Bulletin 91/18**

(84) Etats contractants désignés:
**AT BE CH DE FR GB IT LI LU NL SE**

(56) Documents cités:

**ACTA. BIOLOG. ACAD. SCI. HUNG. (1981), vol. 32 (3-4), pages 195-204; E. VINCZE et al.: "Construction in vitro of R-prime plasmids and their use for transfer of chromosomal genes and plasmids of Rhizobium meliloti"**

**PLASMID, vol. 13, no. 2, 1985, pages 99-105, Academic Press Inc., US; M.F. HYNES et al.: "The development of plasmid-free strains of Agrobacterium tumefaciens by using incompatibility with a Rhizobium meliloti plasmid to eliminate pAtC58"**

(73) Titulaire: **INSTITUT NATIONAL DE LA RECHERCHE AGRONOMIQUE (INRA)**
**145, Rue de l'Université**
**F-75341 Paris Cédex 07(FR)**

(72) Inventeur: **Tepfer, David**
**9, Passage de la Main d'Or**
**F-75011 Paris(FR)**
Inventeur: **Goldmann, Arlette**
**57, rue de Versailles**
**F-92410 Ville d'Avray(FR)**
Inventeur: **Rosenberg, Charles**
**68, rue des Cigognes**
**F-31520 Ramonville-Saint Agne(FR)**

(74) Mandataire: **Warcoin, Jacques et al**
**Cabinet Régimbeau 26, avenue Kléber**
**F-75116 Paris(FR)**

JOURNAL OF BACTERIOLOGY, vol. 149, no. 1, 1982, pages 114-122; H.M. MEADE et al.: "Physical and genetic characterization of symbiotic and auxotrophic mutants of Rhizobium meliloti induced by transposon Tn5 mutagenesis"

JOURNAL OF BACTERIOLOGY, vol. 158, no. 2, 1984, pages 580-589; G. SELVARAJ et al.: "Transposon Tn5 specifies streptomycin resistance in Rhizobium spp"

NATURE, vol. 289, no. 5793, janvier 1981, pages 85-88, Macmillan Journals Ltd., Chesham, Bucks, GB; G.B. RUVKUN et al.: "A general method for site-directed mutagenesis in prokaryotes"

**Description**

La rhizosphère, ou la terre entourant les racines de plante, est riche en microorganismes qui créent des relations avec des plantes, ayant des effets pouvant aller du bénéfique au nuisible.

Le contrôle par génie génétique des relations écologiques entre plantes et microorganismes rhizosphériques pourrait aboutir à des améliorations agronomiques certaines.

La composition de cette population de microorganismes varie selon les sols, l'espèce de la plante, selon l'état physiologique de la plante, etc. et est dirigée en partie, semble-t-il, par les échanges métaboliques entre les parties souterraines des plantes et les organismes habitant la rhizosphère. Certains de ces échanges pourraient impliquer des médiateurs métaboliques spécifiques.

Dans cette hypothèse de spécificité métabolique, on a découvert que les racines excrètent des composés riches en énergie, qui semblent réfractaires au catabolisme par la majorité des microorganismes de la rhizosphère, mais procurent une source d'énergie exclusive pour sélectionner les microorganismes possédant l'information nécessaire pour leur catabolisme. La plante semble contrôler l'excrétion de tels médiateurs chimiques et, de cette façon, contrôle les nombres et genres de microorganismes peuplant sa rhizosphère en créant, en quelque sorte, une pression de sélection. En effet, de telles substances libérées par les plantes en quantité suffisante pourraient procurer des sources de nutrition sélectives aux microorganismes possédant l'information génétique nécessaire à leur catabolisme.

Ces médiateurs métaboliques riches en énergie et qui sont excrétés et produits abondamment par les racines des plantes dans leur rhizosphère seront appelés ci-après du nom générique de "rhizosphérines". Bien que dans ce qui suit on se soit plus particulièrement préoccupé de décrire deux rhizosphérines particulières, à savoir les calystégines 1 et 2 qui ont été observées dans la rhizosphère de Calystegia sepium, il est bien entendu que d'autres types de rhizosphérines pourront être trouvés par des méthodes équivalentes dans la rhizosphère d'autres plantes.

On concevra l'importance de cette mise en évidence lorsque l'on saura que la capacité à cataboliser ces médiateurs riches en énergie est, en général, portée par des plasmides qui peuvent, moyennant des techniques mises en oeuvre dans le domaine du génie génétique, être transférés à des microorganismes particuliers et préalablement déterminés.

C'est pourquoi la présente invention concerne des vecteurs de transformation de microorganismes de la rhizosphère, en particulier des microorganismes à action rhizosphérique favorable, caractérisés en ce qu'ils confèrent auxdits microorganismes transformés la capacité de cataboliser une rhizosphérine particulière.

Bien entendu, le gène codant pour le catabolisme d'une rhizosphérine et qui sera inséré dans le vecteur de transformation en cause sera prélevé sur une souche catabolisant ladite rhizosphérine.

De façon générale, il semble que le gène codant pour ce catabolisme provient d'un plasmide de ladite souche.

Ainsi, on a pu mettre en évidence, en particulier pour les calystégines qui seront décrites en détail dans les exemples qui suivent, le fait que la capacité à cataboliser ces calystégines était portée par un plasmide d'une souche de Rhizobium, en particulier la souche de Rhizobium meliloti 41. Ledit plasmide sera appelé ci-après "plasmide pRme41a".

Il est bien entendu que d'autres rhizosphérines pourront être mises en oeuvre, de même que les plasmides ou fragments de gène qui sont responsables de leur catabolisme.

Les vecteurs selon la présente invention comporteront, outre le gène codant pour le catabolisme de la rhizosphérine, des éléments permettant la transformation des microorganismes que l'on désire transformer et assurant l'expression dudit gène dans ledit microorganisme.

Par exemple, il conviendra d'introduire dans le vecteur tout ou partie d'un marqueur génétique, par exemple tout ou partie d'un transposon, si cela est nécessaire, tel que le transposon Tn5. Il pourra également être nécessaire d'utiliser un vecteur présentant un large spectre d'hôtes de façon à pouvoir l'utiliser pour transformer différents types de microorganismes à activité rhizosphérique favorable.

Outre ces vecteurs, la présente invention concerne, bien entendu, les souches transformées par ces vecteurs, quelle que soit la technique de transformation mise en oeuvre dans la mesure où celle-ci permet d'insérer et de rendre actif le vecteur dans ladite souche. A titre d'exemple, les microorganismes qui pourront être transformés pourront être choisis dans les genres Azospirillum, Agrobacterium et Rhizobium.

En particulier, il pourra s'agir de la transformation de souches de Rhizobium meliloti ou d'Agrobacterium tumefaciens.

Les souches de microorganismes rhizosphériques ainsi transformées sont plus particulièrement utilisables à titre d'agent à action rhizosphérique favorable que l'on peut mettre en oeuvre dans le domaine de l'agriculture. En particulier ces souches transformées pourraient permettre, dans certains cas, d'écarter les problèmes de compétition que l'on rencontre avec les souches que l'on tente d'introduire artificiellement dans la rhizosphère de certaines plantes et qui ne sont pas capables d'entrer en compétition avec les souches autochtones.

Des souches de microorganismes transformées et capables d'assimiler les rhizosphérines se trouvant au voisinage des racines des plantes en cause devraient pouvoir résister plus facilement et être plus compétitives.

L'invention concerne aussi le procédé de transformation des microorganismes, caractérisé en ce qu'il comporte l'utilisation d'un gène catabolisant une rhizosphérine, ainsi que ledit gène en tant que moyen dudit procédé.

D'autres caractéristiques et avantages de la présente invention apparaîtront à la lecture des exemples ci-après, à l'aide des figures qui représentent :

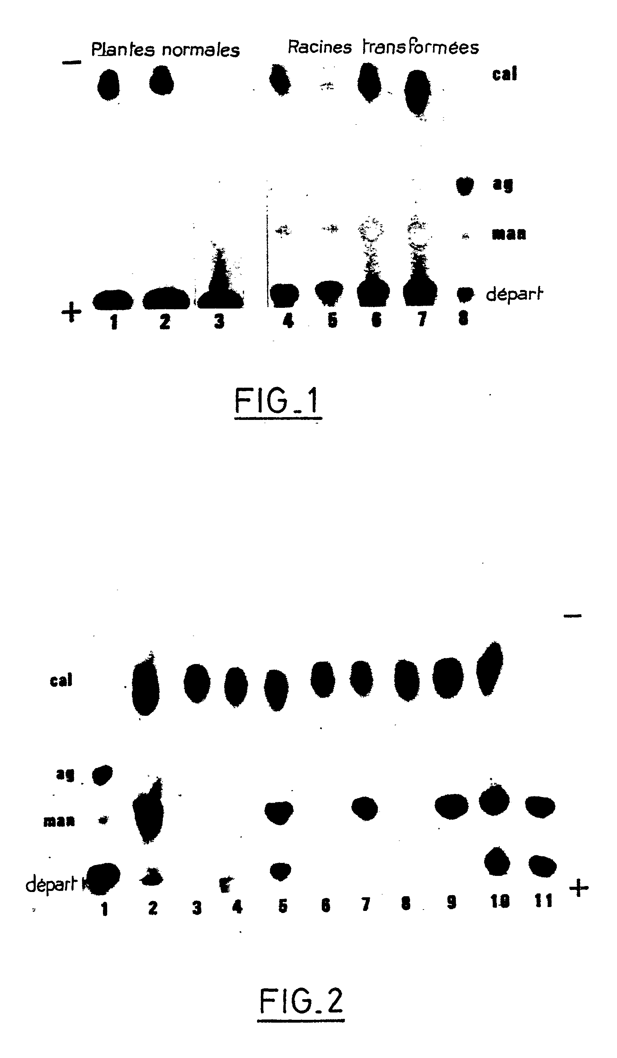

. Pour la figure 1 à gauche, les calystégines 1 et 2 extraites des racines, rhizomes et feuilles de plantes C. sepium normales, poussées en pots :
 - ligne 1 = 5 mg (poids frais) de racines d'une plante normale poussée en pot ;
 - ligne 2 = 5 mg (poids frais) de rhizomes ;
 - ligne 3 = 5 mg (poids frais) de feuilles.
. Pour la figure 1 à droite, les calystégines 1 et 2 extraites de racines transformées cultivées in vitro ou accumulées dans le milieu de culture après 21 jours de culture :
 - ligne 4 = extrait représentant 5 mg de racines transformées ;
 - ligne 5 = 5 μl de milieu employé après 21 jours de culture ;
 - ligne 6 = concentré de 62 μl de milieu employé ;
 - ligne 7 = concentré de 125 μl de milieu ;
 - ligne 8 = migration témoin (extrait représentant 5 mg de lignée de tumeur A66) (1).

(Départ = point de dépôt des échantillons ; man = mannopine ; ag = agropine ; cal = calystégines 1 et 2).

On peut détecter les extraits de calystégine dans les racines de plantes normales poussées in vitro, mais moins que dans les racines transformées en culture.

. Pour la figure 2, la spécificité catabolique des bactéries de la rhizosphère à l'égard des calystégines. Des résultats sont donnés ici à titre d'exemples :
 ligne 1 = extrait standard de lignée tumorale A66 (1),
 ligne 2 = controle sans bactérie,
 ligne 3 = A. rhizogenes A4,
 ligne 4 = A. rhizogenes 8196,
 ligne 5 = A. tumefaciens C58,
 ligne 6 = A. tumefaciens Bo542,
 ligne 7 = A. tumefaciens C58 Cl,

ligne 8 = A. tumefaciens B6-806,
ligne 9 = A. tumefaciens T37,
ligne 10 = R. meliloti L530, et
ligne 11 = R. meliloti 41.

(départ = le point de dépôt des échantillons ; man = mannopine ; ag = agropine ; cal = calystégines 1 et 2).

Toutes les souches testées, que ce soit montré ou non, sont négatives pour le catabolisme de la calystégine, à l'exception de R. meliloti souche 41 qui débarrasse le milieu de toute calystégine détectable (ligne 11). Un extrait de R. meliloti 41 lysé de cette culture après incubation ne contient pas de calystégine, ce qui montre que la dégradation, non seulement l'assimilation, à pris place. Ainsi qu'attendu, A.rhizogenes souches A4 et 8196, et A. tumefaciens souches Bo 542 et B6-806 sont positifs pour le catabolisme de la mannopine. De façon inattendue, les souches V7 et L530 de R. meliloti ont catabolisé la mannopine.

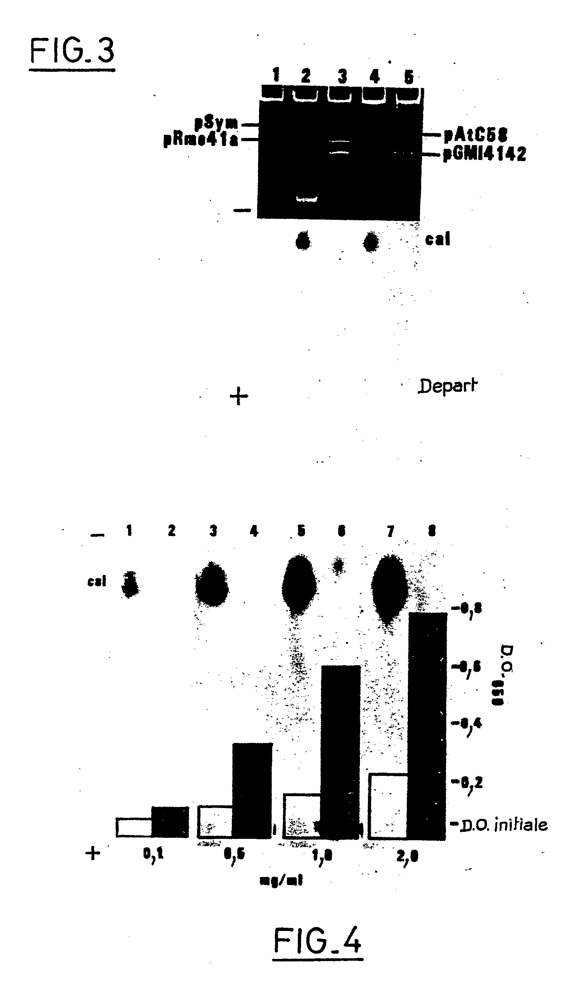

. Pour la figure 3, en haut, les profils plasmidiques des souches bactériennes utilisées pour montrer que le phénotype Cac + est corrélé avec la présence de pRme41a :
 Ligne 1 = R.meliloti 41 type sauvage portant pRme41a aussi bien qu'un mégaplasmide.
 Ligne 2 = GMI13, un dérivé de la souche 41, débarassé du pRme41a.
 Ligne 3 = GMI17, souche 41 (pRme41a: :Tn5) contenant pGMI4142, un dérivé RP4 utilisé pour mobiliser pRme 41a.
 Ligne 4 = C58Cl, un A. tumefaciens débarrassé du plasmide Ti, mais ayant encore pAtC58, un plasmide cryptique.
 Ligne 5 = GMI9019, C58Cl dans lequel pGMI4142 et pRme4: : Tn5 ont été introduits, causant la perte de pATC58.
. Pour la figure 3, en bas, les tests cataboliques utilisant la calystégine extraite des rhizomes de plantes normales, montrant que les fonctions Cac sont codées dans pRme41a. Les mêmes souches que dans la division du haut, voir les méthodes correspondant à la figure 2. Les tests cataboliques utilisant la calystégine à partir des racines transformées donnent des résultats identiques (par exemple seulement les souches contenant pRme41a sont Cac+).

(Départ = point de dépôt des échantillons ; cal = calystégines 1 et 2).

. Pour la figure 4, la croissance calystégine-dépendante : R. meliloti du type sauvage et la même bactérie débarrassée de pRme41a (souche GMI13) ont poussé dans un milieu SM avec les calystégines 1 et 2 comme seules sources de carbone et d'azote. La croissance est contrôlée par $DO_{650}$, et la

dégradation de calystégine est testée par électrophorèse sur papier haut voltage et coloration à l'argent du surnageant après trois jours de culture (voir les méthodes correspondant à la figure 2). L'histogramme de croissance est surimprimé sur l'électrophorétogramme. Les barres vides = R. meliloti débarrassé de pRme41a (souche GMI13) ; les barres pleines = souche 41 type sauvage contenant pRme41a.

Les calystégines 1 et 2 sont partiellement purifiées ainsi que décrit dans la figure 2, suivi par une purification supplémentaire en utilisant la filtration sur gel sur Trisacryl GS05 et électrophorèse sur papier haut voltage .
(Départ = dépôt des échantillons ;
cal = calystégines 1 et 2).

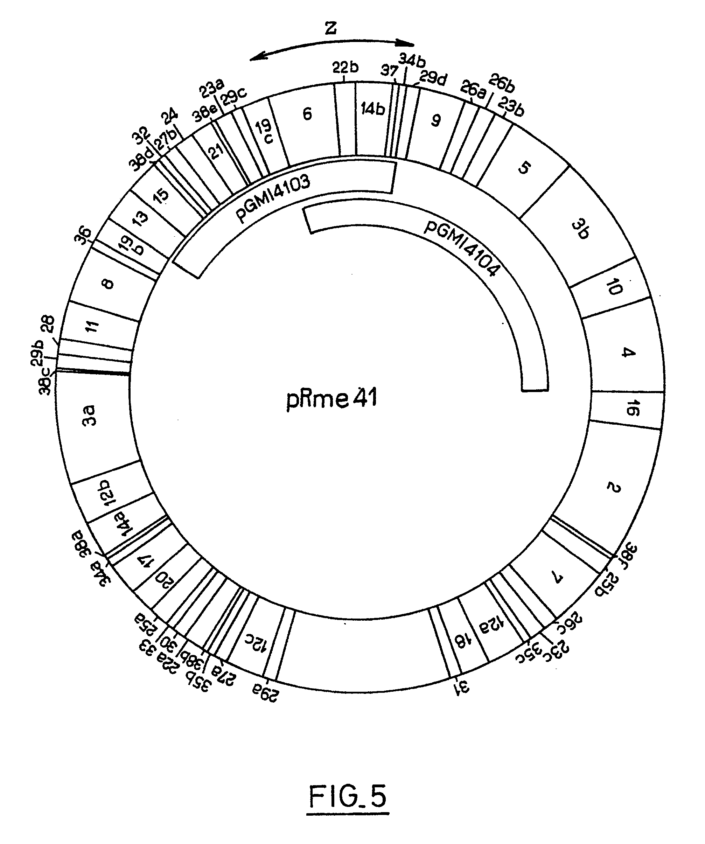

. Pour la figure 5, la carte des sites de restriction HindIII.
z = région d'insertion d'un Tn5 entrainant un phénotype Cac⁻.

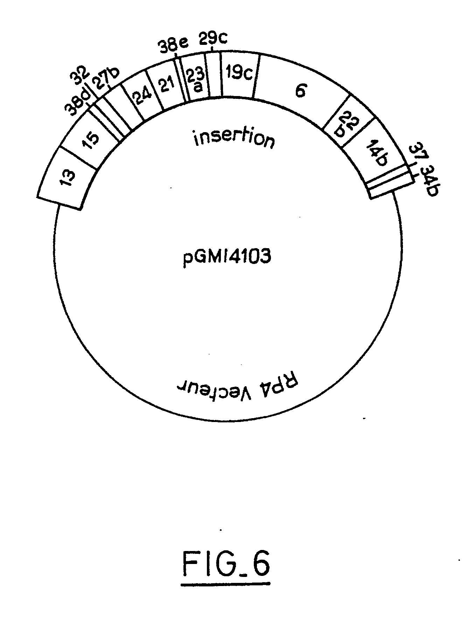

. Pour la figure 6, la carte d'un plasmide à large spectre d'hôte, dérivé de RP4, qui a la propriété de conférer le caractère de catabolisme des calystégines, grâce à un fragment du pRme41.

## METHODES

### 1) L'extraction, l'électrophorèse haut voltage et la coloration à l'argent (figure 1)

Le tissu de la plante est homogénéisé dans HCl 0,1 N (1 $\mu$l/$\mu$gde tissu).
Un minimum de 10 mg est nécessaire.
L'homogénat est chauffé pendant 3 minutes dans un bain d'eau bouillante, et clarifié par centrifugation pendant 5 minutes dans une microfugeuse Eppendorf. Un échantillon de 5 $\mu$l de surnageant est repéré sur papier Whatmann 3MM et soumis à 3 000 volts pendant 15 minutes dans un tampon consistant en acide formique/acide acétique/eau (30/60/910)pH = 1,9.

Après séchage, l'électrophorétogramme est coloré au nitrate d'argent par une première immersion dans une solution d'AgNO₃ (4g dissous dans 20 ml d'eau, dilué à 1 000 ml avec de l'acétone), puis séché et développé dans une seconde solution préparée par dissolution de 5g de NaOH dans 100 ml d'eau, et dilution à 1 000 ml avec 95 % d'EtOH.

Après séchage une troisième fois, l'électrophorétogramme est fixé dans un papier photographique fixateur, et lavé. Les étalons agropine et mannopine sont extraits de la même manière à partir d'une lignée de tumeur de tabac transformée par A. tumefaciens souche A66 (1).

### 2) La préparation et l'enrichissement biologiques des extraits avant les tests de catabolisme (figure 2)

Le traitement par Dowex 50, suivi par l'analyse électrophorétique de l'éluat, montre les calystégines 1 et 2 co-purifiées avec la fraction aminoacide. Cette fraction est évaporée à sec, dissoute dans l'eau, stérilisée sur filtre, et ajoutée à une culture de A. Tumefaciens, souche T37, dans la phase de culture logarithmique. Après 40 heures, un aliquot est testé pour la présence de calystégines et d'amino-acides par électrophorèse sur papier haut voltage et coloration au nitrate d'argent et à la ninhydrine. La souche T37 est débarrassée de tout composé positif détectable à la ninhydrine excepté les calystégines 1 et 2 et la mannopine. Peu ou pas de sucres neutres sont restés après cette purification biologique. Après la suppression des bactéries par centrifugation et filtration, les extraits de racine et de rhizome sont à nouveau traités sur Dowex 50 pour supprimer les sels du milieu, et leur pureté est établie par électrophorèse et coloration.

### 3) Les tests cataboliques (figure 2)

200 $\mu$l des mélanges réactionnels incluant des extraits de racine transformés et enrichis contenant approximativement 20 $\mu$g de calystégines 1 et 2, 20 $\mu$g de mannopine, des sels minéraux du milieu SM, et des bactéries à une concentration initiale de 0,5 OD₆₅₀. Après 40 heures à 28° les bactéries sont supprimées par centrifugation et le surnageant est évaporé à sec. Il est redissous dans 20 $\mu$l d'eau, et un aliquot de 5 $\mu$l de cette solution est soumis à une électrophorèse sur papier haut voltage, suivie par une coloration à l'argent (voir les méthodes correspondant à la figure 1) pour un essai de la dégradation de la calystégine. 34 souches des bactéries sont testées : Agrobacterium rhizogenes souches A4, 8196 ; Agrobacterium tumefaciens souches C58, C58C1, T37, B6-806, Bo-542 ; Rhizobium meliloti 145, S26, 1322, 102F51, V7, CC169, 2011, 41 ; Rhizobium leguminosarum souches 248, PX31, RBL1 ; Rhizobium trifolii souches RCRO402, RCRO403, 32 ; Rhizobium phaseoli souche 8002 ; Azospirillum lipoferum (2 souches non nommées mobiles et non-mobiles), souche Br17 ; Azospirillum brasilense souches CD1, R.07, Sp7, K67, KR77 ; Klebsiella oxytoca, Pseudomonas paucimobilis, Beijerinckia sp (souche V), Enterobacter cloacae (souche C)

## 4) La génétique bactérienne (figure 3)

pRme41a est ciblé avec le transposon Tn5, qui confère la résistance à la kanamycine (Km$^r$) (14). Le plasmide résultant, pRme41a: :Tn5, renommé pGMI59, est mobilisé dans A. tumefaciens C58CI, utilisant un plasmide prime RP4, pGMI4142, consistant en un fragment de pRme41a cloné in vitro dans RP4 (15). Ce RP4 prime est introduit dans R.meliloti41 contenant pGMI59. La souche résultante est utilisée comme un donneur et un Rif$^r$ Sm$^r$ dérivé de A. tumefaciens C58CI comme un receveur. Les transconjugants sont sélectionnés en présence de Km (30 $\mu$g/ml) et les donneurs sont sélectionnés par comptage en présence de Rif (100 $\mu$g/ml) et Sm (100 $\mu$g/ml).

## EXEMPLE 1

A l'origine, on a sélectionné 100 espèces de plantes (représentant 23 familles) pour leurs métabolites secondaires. énergétiques trouvés dans les racines et les feuilles, à partir d'extraits bruts de plantes que l'on a soumis à une électrophorèse sur papier suivie d'une coloration au nitrate d'argent permettant de visualiser les composés contenant du sucre (2).

Deux substances chargées positivement ont été observées dans les racines et rhizomes de Calystegia sepium (Gloire du matin) et n'ont été trouvées que rarement et à des concentrations faibles dans les feuilles et les tiges (figure 1 à gauche).

L'excrétion de ces substances a été observée à partir des racines de plantes ayant poussé dans des conditions aseptiques.

Ces deux substances ont été appelées "calystégines 1 et 2". La calystégine 2 a pour formule brute $C_7H_{13}NO_4$.

D'autres composés ayant des propriétés similaires ont été trouvés dans les feuilles et les racines de Convolvulus arvensis (3), Atropa belladonna et Beta vulgaris.

La méthode de détection des calystégines ayant été la même que celle utilisée pour l'agropine et la mannopine (substances découvertes initialement dans les tumeurs de plantes induites par Agrobacterium tumefaciens) (1), un extrait de lignée de la tumeur de tabac,utilisé à l'origine pour décrire l'agropine et la mannopine, a servi d'étalon externe pour les estimations de contenu en calystégine.

La comparaison entre l'agropine et la calystégine sur la base du poids à l'état frais montre que ces deux substances sont présentes à des concentrations similaires. L'agropine dans la lignée tumorale a été estimée représenter environ 7 % du poids sec (4)

On peut conclure que la synthèse de calystégine représente un engagement anabolique majeur de la part de la plante.

On crée des cultures d'organes de racines de C. sepium dans le but d'obtenir une source continue, uniforme et abondante de calystégines et de déterminer si elles sont excrétées.

Les tentatives de cultures de racines excisées à partir de graines germées in vitro ayant donné des résultats médiocres, on utilise la transformation génétique par Agrobacterium rhizogenes, souche 8196, qui consiste à insérer une partie de l'ADN plasmidique de cette souche dans le génome de la plante pour induire la formation de racines adventives sur les tiges de C. sepium.

Ces racines contiennent une partie (T-DNA) du plasmide Ri (induisant les racines) qui confère aux racines la possibilité de croître rapidement en culture (5, 6, 7).

On crée ainsi une culture continue de racines de C. sepium qui présente une dominance apicale réduite (ramification fréquente) et une vitesse d'élongation aux extrémités de 1 à 2 cm par jour.

Des quantités de l'ordre du kilogramme ont été produites dans un milieu liquide.

Comme dans le cas de plantes normales poussant in vitro, une excrétion abondante de calystégines 1 et 2 dans le milieu de culture a été observée en boites de Pétri (figure 1 à droite).

Après trois semaines de culture, 5 $\mu$l de milieu de culture ont été suffisants pour détecter les calystégines, et sur une base pondérale (état frais) leur concentration dans le milieu était plus basse mais du même ordre de grandeur qu'à l'intérieur des racines de la même culture. Ainsi, une partie majeure des calystégines 1 et 2 produite par les racines est excrétée dans le milieu.

Les racines de C. sepium transformées produisent de la mannopine, une substance caractéristique des racines contenant Ri-T-DNA (5, 8).

Le catabolisme de la mannopine est spécifique de certaines souches A. tumefaciens et A. rhizogenes.

La mannopine a été co-purifiée avec les calystégines 1 et 2 et a servi d'étalon interne dans les tests de catabolisme utilisés pour la recherche des bactéries du sol capables de cataboliser les calystégines.

Préalablement aux tests de catabolisme, des extraits de racines transformées (contenant des calystégines et de la mannopine) ou des extraits de rhizomes excisés à partir de plantes normales (contenant seulement des calystégines) ont été partiellement purifiés. Ils contiennent peu ou pas de sucres neutres contaminants.

EXEMPLE 2

Des souches des genres Azospirillum, Agrobacterium et Rhizobium ont été choisies dans la recherche des bactéries du sol capables de dégrader les calystégines parce que ces bactéries ont été décrites en détail des points de vue génétique et écologique (9, 10, 11).

Un total de 34 souches a été testé.

Le catabolisme de la mannopine a été positif pour les souches A. tumefaciens portant les plasmides Ti de type octopine et pour les souches A. rhizogenes ; il a été négatif pour les souches portant les plasmides Ti de type nopaline, confirmant la fiabilité des tests de catabolisme.

Le catabolisme des calystégines 1 et 2 a été positif pour seulement une des souches examinées, Rhizobium meliloti 41 (figure 2).

On désigne ce phénotype catabolique par Cac + (= Calystégine catabolisme).

Dans R. meliloti souche 41, trois plasmides ont été détectés : un plasmide cryptique de 215 kb nommé pRme41a, et deux megaplasmides de plus de 700 kb. Un de ceux-ci, pSym, porte les gènes impliqués dans la nodulation des légumineuses et la fixation de l'azote (12, 13, 14).

Après marquage du plasmide cryptique Rme41a par échange de marqueur, en utilisant le transposon Tn5, on a isolé un dérivé de R. meliloti 41 débarrassé de pRme41a. Cette souche est Cac⁻ ; ce qui indique que pRme41a porte le gène (s) Cac.

Après marquage du plamide cryptique pRme41a en utilisant le transposon Tn5, on observe l'auto-transfert de pRme41a : Tn5 obtenu à d'autres souches R.meliloti avec une basse fréquence ($10^{-6}$) mais pas à Agrobacterium.

Afin de permettre une étude complète dans différents hôtes, on utilise un autre plasmide mobilisateur à large spectre d'hôtes. Pour ce faire, on utilise un plasmide dérivé du plasmide RP4 : le plasmide pGMI4142.

La souche hôte utilisée est A. tumefaciens C58C1 (débarrassée de son plasmide Ti).

Alors que cette souche C58C1 est Cac⁻, les transconjuguants de C58C1 (pRme41a: :Tn5) (pGMI4142) catabolisent les calystégines 1 et 2, montrant que les gènes responsables du catabolisme de la calystégine (cac) sont localisés sur ce plasmide (figure 3).

EXEMPLE 3

Les essais suivants ont eu pour but d'objectiver l'activité du vecteur construit à l'exemple 2.

Puisque la souche C58C1 de contrôle (pGMI4142) était Cac⁻, les souches C58C1 (pRme41a: :Tn5) (pGMI4142) transconjuguées ont catabolisé les calystégines 1 et 2, montrant que les gènes responsables du catabolisme de la calystégine (cac) sont localisés sur ce plasmide.

Le plasmide pRme41a étant incompatible avec pAtC58, un plasmide cryptique de 410 kb de C58C1, les transconjugués des souches C58C1 (pRme41a: :Tn5) sont de cette façon débarrassés du plasmide pAtC58 (15).

Ce n'est pas l'enlèvement du pAtC58 qui rend la souche C58C1 (pRme41a: :Tn5) Cac⁺, puisque C58C1 débarrassé de pAtC58 est Cac⁻.

De plus, la souche 41 débarrassée du pRme41a est Cac⁻, ce qui confirme que pRme41a porte les gènes cac et indique que les copies fonctionnelles de ces gènes ne sont pas présentes ailleurs.

EXEMPLE 4 :

Les bactéries Cac⁻ et Cac⁺ ont été testées pour leur capacité à pousser dans un milieu pauvre en nutriments, contenant les calystégines 1 et 2 comme seules sources de carbone et d'azote, extraites comme décrit ci-dessus, puis purifiées par gel-filtration et électrophorèse préparative sur papier.

Après 3 jours dans la culture, la croissance a été stimulée plus de 3 fois chez les bactéries Cac⁺ par rapport aux bactéries de contrôle Cac⁻.

De façon plus générale, la croissance chez les bactéries Cac⁺ a été stimulée par rapport aux bactéries de contrôle Cac⁻, proportionnellement à la concentration en calystégine dans le milieu. Cette croissance différentielle était accompagnée par l'élimination des calystégines du milieu par les bactéries Cac⁺ (figure 4).

Bien que le Demandeur ne souhaite pas être limité par une explication scientifique, on pense que la faible croissance observée chez les contrôles est due à des impuretés dans l'extrait de calystégine ou à une lente autodégradation de la calystégine.

On a montré que, dans les conditions de laboratoire, les calystégines ont les propriétés de médiateurs chimiques fonctionnels naturels. Elles sont synthétisées en abondance par les organes souterrains ; elles sont libérées dans l'environnement des racines ; et elles procurent une source exclusive de carbone et d'azote aux bactéries possédant les gènes de leur catabolisme. D'autres composants d'exsudats de racine pourraient avoir des caractéristiques similaires, et pourraient être impliqués dans la médiation des relations écologiques dans la rhizosphère.

On propose de donner à de telles substances le nom général de rhizosphérines. La synthèse de rhizosphérine (par exemple Cas) et leur catabolisme (par exemple Cac) peuvent être utilisés pour promouvoir la survie des bactéries du sol génétiquement améliorées ou pour créer de nouvelles associations plante-microorganisme. Un tel contrôle de l'écologie de la rhizosphère ne peut que conduire à des améliorations significatives dans la culture de plantes.

**Revendications**

1.  Procédé de transformation d'un microorganisme pour lui permettre d'accéder à la rhizosphère d'une plante régulant sa flore microorganique rhizosphérique par émission de rhizosphérine(s) dont le catabolisme par ladite flore microorganique rhizosphérique est codé par un gène porté par un plasmide, caractérisé par le fait que :
    a) l'on identifie les rhizosphérines émises par la plante, le gène codant et le plasmide portant ce dernier ;
    b) on synthétise un vecteur de transformation ;
    c) on fait agir ledit vecteur de transformation sur ledit microorganisme.

2.  Procédé selon la revendication 1, caractérisé par le fait que ladite ou lesdites rhizosphérines sont susceptibles d'être catabolisées par une souche de Rhizobium, de préférence par une souche de Rhizobium meliloti.

3.  Procédé selon l'une des revendications 1 et 2, caractérisé par le fait que ledit plasmide provient d'une souche de Rhizobium meliloti.

4.  Procédé selon les revendications 1 à 3, caractérisé par le fait que ledit plasmide est le plasmide pRme41a.

5.  Procédé selon l'une des revendications 1 à 4, caractérisé par le fait que la, ou les, rhizosphérine(s) sont choisies parmi les calystégines.

6.  Procédé selon l'une des revendications 1 à 5, caractérisé en ce qu'on utilise un vecteur de transformation comportant outre un gène codant la capacité de cataboliser une rhizosphérine, tout ou partie d'un marqueur génétique tel qu'un transposon.

7.  Procédé selon l'une des revendications 1 à 6, caractérisé en ce qu'on utilise un vecteur de transformation dérivé du plasmide RP4.

8.  Souche de bactéries, caractérisée par le fait qu'elle est transformée par le procédé selon l'une des revendications 1 à 5.

9.  Souche selon la revendication 8, caractérisée par le fait que les souches soumises à la transformation sont choisies parmi les genres Agrobacterium et Rhizobium.

10. Souche selon les revendications 8 et 9, caractérisée par le fait que la souche soumise à transformation est choisie parmi les souches Rhizobium meliloti et Agrobacterium tumefaciens.

11. Application des souches selon l'une des revendications 8 à 10 à titre d'agent à action rhizosphérique favorable dans l'agriculture.

12. Gènes catabolisant une rhizosphérine, en tant que moyen du procédé selon l'une des revendications 1 à 7.

**Claims**

1.  A process for transforming a micro-organism to give it access to the rhizosphere of a plant controlling its rhizosphoric micro-organic flora by emission of rhizopherin(s), the catabolism of which by the said rhizospheric micro-organic flora is coded by a gene carried by a plasmid, characterised in that:
    a) the rhizospherins emitted by the plant, the coding gene and the plasmid carrying the gene are identified:
    b) a transformation vector is synthesized, and
    c) the transformation vector is caused to act on the said micro-organism.

2.  A process according to claim 1, characterised in that the rhizospherin or rhizospherins are capable of being catabolised by a strain of Rhizobium, preferably a strain or Rhizobium meliloti.

3.  A process according to claim 1 or 2, characterised in that the plasmid originates from a strain of Rhizobium meliloti.

4.  A process according to claims 1 to 3, characterised in that the plasmid is plasmid pRme41a.

5.  A process according to any of claims 1 to 4, characterised in that the rhizospherin or rhizospherine are chosen from among calystegins.

6. A process according to any of claims 1 to 5, characterised by use of a transformation vector comprising all or part of a genetic marker such as transposon in addition to a gene coding the capacity to catabolise a rhizospherin.

7. A process according to any of claims 1 to 6, characterised by use of a transformation vector derived from plasmid RP4.

8. A bacteria strain, characterised in that it is transformed by the process according to any of claims 1 to 5.

9. A strain according to claim 8, characterised in that the strains subjected to transformation are chosen from among the genera Agrobacterium and Rhizobium.

10. A strain according to claim 8 or 9, characterised in that the strain subjected to transformation is chosen from among the strains Rhizobium meliloti and Agrobacterium tumefaciens.

11. Use of the strains according to any of claims 8 to 10 as a rhizospheric agent which is advantageous in agriculture.

12. Genes catabolising a rhizospherin, as a means in the process according to any of claims 1 to 7.

**Ansprüche**

1. Verfahren zur Transformation eines Mikroorganismus, um ihm Zugang zur Rhizosphäre einer Pflanze zu verschaffen, die ihre mikroorganische Rhizosphären-Flora durch Emission von Rhizospherin(en) reguliert, deren Katabolismus durch die genannte mikroorganische Rhizosphären-Flora durch ein von einem Plasmid getragenes Gen kodiert ist, dadurch gekennzeichnet, daß man
   a) die von der Pflanze emittierten Rhizospherine, das kodierende Gen und das letzteres tragende Plasmid identifiziert;
   b) einen Transformationsvektor synthetisiert; und
   c) den Transformationsvektor mit dem Mikroorganismus reagieren läßt.

2. Verfahren nach Anspruch 1, dadurch gekennzeichnet, daß das (die) Rhizospherin(e) durch einen Stamm von Rhizobium, vorzugsweise einen Stamm von Rhizobium meliloti, katabolisiert werden kann (können).

3. Verfahren nach einem der Ansprüche 1 und 2, dadurch gekennzeichnet, daß das Plasmid aus einem Stamm von Rhizobium meliloti stammt.

4. Verfahren nach den Ansprüchen 1 bis 3, dadurch gekennzeichnet, daß es sich bei dem Plasmid um das Plasmid pRme41a handelt.

5. Verfahren nach einem der Ansprüche 1 bis 4, dadurch gekennzeichnet, daß das (die) Rhizospherin(e) ausgewählt wird (werden) unter den Kalysteginen.

6. Verfahren nach einem der Ansprüche 1 bis 5, dadurch gekennzeichnet, daß man einen Transformationsvektor verwendet, der außer einem Gen, das kodiert für die Fähigkeit, ein Rhizospherin zu katabolisieren, einen genetischen Marker, wie ein Transposon, ganz oder einen Teil davon aufweist.

7. Verfahren nach einem der Ansprüche 1 bis 6, dadurch gekennzeichnet, daß man einen Transformationsvektor verwendet, der aus dem Plasmid RP4 stammt.

8. Bakterienstamm, dadurch gekennzeichnet, daß er nach dem Verfahren nach einem der Ansprüche 1 bis 5 transformiert worden ist.

9. Stamm nach Anspruch 8, dadurch gekennzeichnet, daß die der Transformation unterworfenen Stämme ausgewählt werden aus den Genus Agrobacterium und Rhizobium.

10. Stamm nach den Ansprüchen 8 und 9, dadurch gekennzeichnet, daß der der Transformation unterworfene Stamm ausgewählt wird aus den Stämmen Rhizobium meliloti und Agrobacterium tumefaciens.

11. Verwendung der Stämme nach einem der Ansprüche 8 bis 10 als in der Landwirtschaft vorteilhaftes Agens mit rhizosphärischer Wirkung.

12. Gene, die ein Rhizospherin katabolisieren, als Mittel zur Durchführung des Verfahrens nach einem der Ansprüche 1 bis 7.

FIG.1

FIG.2

# FIG.3

pSym
pRme41a

pAtC58
pGMI4142

cal

+

Depart

cal

D.O.650

-0,8

-0,6

-0,4

-0,2

-D.O. initiale

+    0,1    0,5    1,0    2,0

mg/ml

# FIG.4

FIG.5

FIG_6